# EUROPEAN PATENT APPLICATION

(11) **EP 1 525 873 A1**
(43) Date of publication of application: **27.04.2005**
(21) Application number: 03024276.2
(22) Date of filing: 23.10.2003
(51) Int. Cl.: A61J 1/00, B65D 83/00

(54) **Container with septum member**

(71) Applicant: NOVO NORDISK A/S, 2880 Bagsvaerd (DK)
(72) Inventor: Sparholt, Philip Albert, 2765 Smorum (SE); Winkel Ethelfeld, Erik, 1360 Copenhagen (DK)
(74) Representative: Kristoffersen, Bo

(57) **Abstract**

The present invention provides a container adapted to contain a fluid and comprising a septum of a needle-penetratable self-sealing material welded to an outer surface of the container. In a further aspect the invention provides a reservoir device comprising a flexible reservoir with a septum member, and a needle having a generally straight inlet portion adapted to be arranged in fluid communication with the reservoir outlet means, wherein the needle is arranged to penetrate the septum member at an inclined angle relative thereto. In a yet further aspect the invention provides a reservoir device comprising a housing and a flexible reservoir with an outlet means mounted on a surface of the reservoir, wherein the outlet means is mounted to the housing.

## Description

The present invention generally relates to a container for the storage of fluids, the container comprising access means allowing the interior of the container to be accessed by a fluid conducting means such as a penetrating needle member. The container may be designed to contain in particular medical liquid products such as drugs, drug solutions, infusion solutions, parenteral solutions, dialysis solutions, perfusion solutions, chemical and alimentary liquids, human blood and its fractions, and the like. The container may also be used for other purposes, e.g. calibration liquids for analytical equipment.

### BACKGROUND OF THE INVENTION

In the disclosure of the present invention reference is mostly made to liquids in the form of medical liquids as outlined above, however, this is only an exemplary use of the present invention.

Medical liquids are often supplied in containers which can be accessed by means of a hollow needle, the needle typically penetrating a dedicated connection element (or access means) of the reservoir to provide a fluid communication with the interior of the container. The needle access may be used either for withdrawing liquids from the container or for supplying a liquid to the container. For example, when preparing the fluids which are to be administrated to the body of a patient from a given container, it is common that medically effective substances are supplied to a pre-sealed container which is filled with a transport fluid, usually in the form of sodium chloride solution or a glucose solution, the diluted drug then being given to a patient intravenously via an intravenous (IV) infusion set. For this type of use, the container may be provided with a single connection means adapted to be used for both purposes, or the container may comprise two connection means adapted for their respective purposes, e.g. for a larger infusion set outlet needle and a smaller drug injecting needle. The connection element may be adapted to be opened manually to provide an opening, through which a needle subsequently can be inserted, or the pointed needle can be used for penetrating the connection element, which may be of the self-sealing type, e.g. the connection element will seal the container after the needle has been withdrawn.

In case the container is of glass, the connection element will be a separate element which is mounted to the glass container by special means, however, for plastic containers the connection element will typically be formed integrally with the container. One of the most widely used type of plastic containers for medical use is in the form of a flexible infusion bag comprising at a lower portion thereof one or more needle-penetratable connection elements. Such bags are typically formed from flexible foil sheets which are joined to form an internal space. Depending on the actual construction of the bag, the connection element(s) may be arranged either on a surface portion of a foil sheet or may be arranged corresponding to an edge portion of the container. For the latter type, the connection element is typically positioned and held in place between two foil sheets connected to each other by welding.

For both of the above two arrangements, the self-sealing element is normally carried by a tubular member connected to the bag in either of the above two ways. For example, US patent 4,362,158 shows an infusion bag in which a tubular nozzle member is connected to an infusion bag corresponding to a free surface thereof, a self-sealing rubber seal member being mounted on the nozzle.

For some liquids, e.g. certain types of drugs, it is desirable if the elastomeric material from which the seal member normally is manufactured, does not come in contact with drug. To solve this problem, European patent application EP 0 364 783 describes a medicament bottle having an external sealing element held in place by a separate cap member attached to the outer surface of the bottle.

The above containers are relatively large, typically comprising 100 - 1.000 ml of liquid, however, containers which can be accessed by a penetrating needle member is also used for much smaller volumes. For example, certain calibration solutions for calibrating analytical equipment are supplied in small bag-like containers containing a few ml. When properly designed, such small containers may also be used for drug purposes.

### DISCLOSURE OF THE INVENTION

Having regard to the above-identified known containers, it is an object of the present invention to provide a liquid container or reservoir having a self-sealing needle-penetratable access means which can be manufactured in a cost-effective manner. It is a further object of the invention to provide a container which is convenient and safe in use and allows a varity of applications. It is a yet further object of the invention to provide a container which can be used with a varity of liquids. Further objects and advantages will become apparent from the disclosure of the invention and the description of the exemplary embodiments.

More specifically, the present invention provides in a first aspect a closed container adapted to contain a fluid within a cavity formed by walls, comprising a first wall portion formed from a needle-penetratable material, where the wall portion comprises an outer first mounting surface. The container further comprises a septum member (in the following also referred to as septum) formed from a needle-penetratable self-sealing material, the septum member comprising a second mounting surface, wherein the septum member, corresponding to the second mounting surface, has been mounted on the first mounting surface by means of welding together the two surfaces. The material for the first mounting surface may be located solely corresponding to the placement of the septum, or it may cover a portion or the entire exterior surface of the container.

Although the term "self-sealing" will be clear to the skilled person, it should be noted that this is not to be regarded as an absolute term for a given septum but that it will depend upon the intended use for a given container. For example, a given septum will be designed to be self-sealing in connection with needles of a given range of gauges (i.e. diameters) and with a given design for the pointed distal end. Thus, a relatively thin septum adapted to be used with a correspondingly thin needle may not be self-sealing when penetrated by a larger needle. Further, if the container is pressurized above the intended internal pressure, a punctuated septum may leak.

By the term "adapted to contain" is defined that a container in accordance with the invention may be provided in an empty state for subsequent filling, or that it may be filled during manufacture of the container.

If the fluid (liquid or gas) to be contained in the container does not require specific properties for the surrounding walls (e.g. in respect of evaporation, leakage or chemical inertness), the first wall portion may be made from a single layer of material or the container in general may be formed from a single material. If the fluid requires specific properties for the container, the container may be provided with an outer surface generally formed from a first material and an inner surface generally formed from a second material. The wall portion providing the first mounting surface may be of a special construction (e.g. allowing needle-penetration) with the rest of the container wall having different properties.

The container may be a relative rigid container (e.g. a blown bottle) or a more flexible container as a traditional IV bag. For the latter, the container may comprise at least first and second flexible foil (or film) members sealed together to form at least one enclosed cavity for containing the liquid. The foil members may be provided as two or more separate members or as a single member which is folded upon itself. The foil members may be composite laminates of continuous layers including an outer layer and an inner layer. This would allow the outer surface to be optimized for connection of the septum whereas the inner surface may be optimized in respect of container properties in respect of the fluid to be contained. Any laminate referred to in the present application may be a traditional laminate, a co-extrudate or an extrusion-laminate. The walls defining the container may be formed from a single material or single type of laminate, or different materials or laminates may be used for different wall portions of the container.

The foil members may comprise an intermediate layer, the inner layer being formed from a weldable material, which would allow the foil members to be sealed together at least partially by welding corresponding to the peripheries of the bag. In case the outer layer solely provides the mounting surface for the septum, the "intermediate" layer may provide the outer layer for a portion of the container. To allow a contained liquid to be viewed through the container wall, at least a portion thereof may be formed by a transparent or translucent material.

Just as a portion of the outer container wall surface may be optimized for attachment of the septum by welding, also the septum mounting surface may be optimized. For example, the septum may be a laminate comprising two or more layers, the "lower" layer providing the mounting surface. The septum may be welded to the container corresponding to substantially the entire mounting surface, however, it may also be welded using one or more (concentric) circumferential welds.

As the seal member in accordance with the invention is connected directly to an outer wall portion of the container without the need of using additional elements or means (e.g. adhesives) to secure the septum, the septum can be configured to provide additional properties in addition to the sealing properties. For example, a portion of the septum or the entire septum may be configured to flex together with the portion of the container to which it is mounted, this allowing the septum to be bend either during manufacture or during use. To allow this flexibility the septum may be in the form of a relatively thin member or may comprise a relatively thin portion. Further, when the septum is arranged "naked" on the container, it may be accessed from a wide range of angles.

Although the septum may be mounted alone, it may be desirable to provide additional mounting means allowing access means such a tubing to be connected to the container, e.g. as described above with respect to the known IV bags. For example, a tubular connector protruding from the surface of the container may be attached around the septum, e.g. circumferentially. In this way the two components can be attached independently of each other without having the septum to be mounted to the connector, although it may be convenient to attach the two components to the container simultaneously during a welding procedure.

The septum may also be configured to serve additional purposes. For example, in case the container is in the form of a flexible reservoir adapted to be mounted in a drug delivery device, the septum may be used to handle the reservoir during the mounting procedures just as the septum may be adapted to serve as a connecting means between the reservoir and the delivery device, e.g. the septum member may be welded to the delivery device or the septum member and the delivery device may be provided with mating coupling means.

As discussed above, the "naked" septum arrangement may be adapted to allow insertion of a needle at an angle which may differ considerably from perpendicular. Correspondingly, in a second aspect of the invention a device is provided in which a fluid communication between a reservoir and an outlet needle can be established.

More specifically, in the second aspect of the invention a reservoir device is provided, comprising a flexible reservoir adapted to contain a fluid (e.g. being supplied pre-filled to the user or adapted to be filled (and refilled) by the user), the reservoir comprising a first surface and a needle-penetratable outlet means arranged on a first portion of the first surface, and a needle having a generally straight and pointed inlet portion and an outlet, the inlet being adapted to be arranged in fluid communication with the reservoir outlet means. The needle and the reservoir are moveable relative to each from an initial position in which there is no fluid communication therebetween and a connected position in which the needle inlet is arranged in fluid communication with the reservoir through the reservoir outlet means, wherein the needle in the connected position penetrates the reservoir outlet means at an inclined angle relative to the first surface portion.

In preferred embodiments the inclined angle is less than 45 degrees, preferably less than 30 degrees and more preferably less than 15 degrees (with parallel being defined as zero degrees). By introducing the needle at an inclined angle, the reservoir may collapse fully or partially without the needle penetrating the reservoir portion arranged opposite the outlet means.

In an exemplary embodiment the reservoir comprises first and second flexible foil members sealed together to form an enclosed cavity for containing the fluid, the first foil member comprising the first surface, the reservoir having a pouch-like configuration defining a general plane. In case the pouch is formed by two foil members connected to each other at the periphery thereof, a plane through the connected (e.g. welded) portions will typically define a general plane. When such a reservoir is filled, the first surface will have a generally convex configuration relative to the interior of the reservoir and the general plane. When the needle in the connected position is arranged substantially in parallel with such a general plane, the reservoir can be fully collapsed without the needle penetrating the reservoir.

The reservoir outlet means may be in the form of a septum member formed from a needle-penetratable self-sealing material as described above, the septum advantageously being connected by welding. Dependent upon the fluid to be contained in the reservoir, the walls thereof may comprise one or more layers as described above.

In order to help position the needle in parallel with general plane of the reservoir, the reservoir may comprise first and second portions, the first portion being arranged substantially corresponding to the general plane, the second portion being deflected relative thereto in a direction away from the first surface, the reservoir outlet means being arranged on the second portion of the reservoir. In this way the needle may be introduced into the reservoir at an angle corresponding to the degree of deflection.

In an exemplary embodiment the device is in the form of a delivery device (e.g. an infusion device) further comprising a delivery means having an inlet and an outlet, the inlet being adapted to be arranged in fluid communication with the reservoir through the needle, the delivery means being capable of expelling a fluid (e.g. a drug) contained in the reservoir through the outlet of the delivery means. The delivery means may be in the form of a suction pump (e.g. a membrane pump) drawing liquid from a freely collapsible reservoir.

The septum may advantageously be used to provide a connection between the reservoir and a supporting structure.

Correspondingly, in a third aspect of the invention a reservoir device is provided, comprising a housing, a flexible reservoir arranged in the housing and adapted to contain a fluid, the reservoir comprising a first surface and an outlet means arranged on the first surface, wherein the outlet means is mounted to the housing. In this way a secure fixation between the reservoir and the housing is provided without having to interfere with the flexibility of the reservoir, e.g. substantially the entire flexible reservoir apart from the outlet/mounting means may be arranged free to move relative to the housing, this allowing the reservoir to be emptied to a high degree.

Advantageously, the reservoir comprises first and second flexible foil members sealed together to form an enclosed cavity for containing the fluid, the first foil member comprising the first surface, the reservoir having a pouch-like configuration.

In an exemplary embodiment the device further comprises a fluid communication means having an inlet and an outlet, the inlet being adapted to be arranged in fluid communication with the reservoir outlet means, wherein the fluid communication means and the reservoir are moveable relative to each from an initial position in which there is no fluid communication therebetween and a connected position in which the inlet of the fluid communication means is arranged in fluid communication with the reservoir through the reservoir outlet means. As described above, the fluid communication means may be in the form of (or comprise) a pointed needle and the reservoir outlet means may be in the form of a septum member formed from a needle-penetratable self-sealing material.

In an exemplary embodiment, the device further comprises a delivery means adapted to expel a fluid (e.g. a drug) contained in the reservoir through the outlet means of the reservoir. The expelling means may be adapted to force or suck the drug from the reservoir. In the latter case the delivery means may be in the form of a pump having an inlet and an outlet, the inlet being adapted to be arranged in fluid communication with the reservoir through the needle.

The septum and the materials forming the reservoir may be selected in accordance with the above described embodiments. For example, the wall portion providing the first mounting surface may be manufactured from or comprise polyethylene (PE), polypropylene (PP), oriented polypropylene (OPP), amorphous polyester (PET), oriented amorphous polyester (OPET), polyamide (PA) or oriented polyamide (OPA). The septum portion forming the second mounting surface may be manufactured from a thermoplastic elastomeric (TPE) material, e.g. an oleophenic based thermoplastic material or an oleophenic based thermoplastic vulcanisate, or blends thereof. More specifically, the TPE may be a thermoplastic vulcanisate composed of bromo-butyle rubber in a polypropylene matrix (e.g. as sold under the trade name Trefsin), a thermoplastic vulcanisate composed of EPDM rubber in a polypropylene matrix (e.g. as sold under the trade name Santoprene), or a styrene ethylene butylene styrene (SEBS) copolymer based elastomere (e.g. as sold under the trade name Kraiburg). Also TPU, TPE-A or TPE-E may be used.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the exemplary embodiments reference will be made to the use of insulin. Correspondingly, the term "subcutaneous" infusion is meant to encompass any method of transcutaneous delivery to a subject.

Herein the term "insulin" refers to insulin from any species such as porcine insulin, bovine insulin, and human insulin and salts thereof such as zinc salts, and protamin salts as well as active derivatives of insulin, and insulin analogues. The term "active derivatives of insulin", is what a skilled art worker generally considers derivatives, *vide* general textbooks, for example, insulin having a substituent not present in the parent insulin molecule. The term "insulin analogues" refers to insulin wherein one or more of the amino acid residues have been exchanged with another amino acid residue and/or from which one or more amino acid residue has been deleted and/or from which one or more amino acid residue has been added with the proviso that said insulin analogue has a sufficient insulin activity.

The material(s) used to form the reservoir or reservoir devices of the present invention may be selected in accordance with the intended use. Thus it may be required that the material(s) fulfil(s) specified functional requirements such as physical properties for the material after sterilization, chemical requirements for the material after sterilization, and cleanliness. Correspondingly, the material may be sterilizeable using e.g. gamma irradiation, electron beam, steam, or ethylene oxide. The material(s) may further be selected in accordance with one or more of the following requirements: 1) the material must be transparent, 2) the material must provide a good barrier against water evaporation; 3) the material must provide a good barrier against gasses (for example, oxygen and carbon dioxide); 4) the material must provide a good barrier against preservatives (for example, phenol and meta-cresol); 5) the material must provide a good barrier against odors (for example preservatives); 6) the material must be resistant against environmental stress cracking (for example, oils, perfumes); 7) the material must be resistant against flex-crack; 8) the material must have good sealing properties (for example, by welding); 9) the material must not delaminate after sterilization, during processing or storage; 10) the material must not relax significantly during storage and use, 11) the material must not emit substances to the drug which can affect the health and safety of the patient (leachables); 12) the material must have a very low level of extractables; and 13) the material must be compatible with a contained drug formulation. It may further be relevant that the material(s) fulfil(s) certain health and safety requirements, preferably most of or all the requirements mentioned in 1) European Pharmacopoeia (Ph. Eur.) 2002, 4^{th} edition; 2) The United States Pharmacopeia (USP) 25; 3) Japanese Pharmacopeia (JP) XIV; 4) EEC Directive 90/128 + amendments "Relating to plastics materials and articles intended to come into contact with foodstuffs"; 5) Code of federal regulations (CFR) Title 21 Food and Drugs, part 170 - 190; 6) III/9090/90 EN. Plastic Primary Packaging Materials. Note for Guidance; and 7) Guidance for Industry. Container Closure Systems for Packaging Human Drugs and Biologics, Chemistry, Manufacturing, and Controls Documentation. FDA, May 1999.

In respect of laminates the following definitions are used: Co-extrusion covers a process where two or more polymer materials are melded in two or more extruders and co-extruded together through a flat nozzle or systems of flat nozzles and cooled to form the co-extruded foil. Extrusion-lamination covers a process where a feedstock in form of a foil of one material is coated through a flat nozzle or systems of flat nozzles from one or more extruders with one layer or more layers of melted material or materials and then cooled to form the extrusion-lamination foil. "Traditional" lamination covers a process, where two feed stocks of foil materials are joined together by adding a proper adhesive to one foil, followed by addition of the second foil forming the laminated foil. A tie layer is a layer which is placed between two polymer layers with the object of securing that the two layers are joined together.

### BRIEF DESCRIPTION OF THE DRAWINGS

In the following the invention will be further described with references to the drawings, wherein
fig. 1 shows a side view of an infusion bag,
fig. 2 shows a cross-sectional view along line II-II of fig. 1,
fig. 3 shows a cross-sectional view of an alternative connecting means for an infusion bag,
fig. 4 shows a perspective view of a drug delivery device,
fig. 5 shows a schematic representation of a membrane pump,
fig. 6A shows a perspective view of a flexible reservoir,
fig. 6B shows a side view of the reservoir shown in fig. 6A,
fig. 6C shows an upper view of the reservoir shown in fig. 6A,
fig. 7A shows a side view of a reservoir with a needle inserted,
fig. 7B shows an upper perspective view of the reservoir of fig. 7A, and
fig. 7C shows a lower perspective view of the reservoir of fig. 7A.

In the figures like structures are identified by like reference numerals.

### DESCRIPTION OF EXEMPLARY EMBODIMENTS

When in the following terms as "upper" and "lower", "right" and "left", "horizontal" and "vertical" or similar relative expressions are used, these only refer to the appended figures and not to an actual situation of use. The shown figures are schematic representations for which reason the configuration of the different structures as well as there relative dimensions are intended to serve illustrative purposes only.

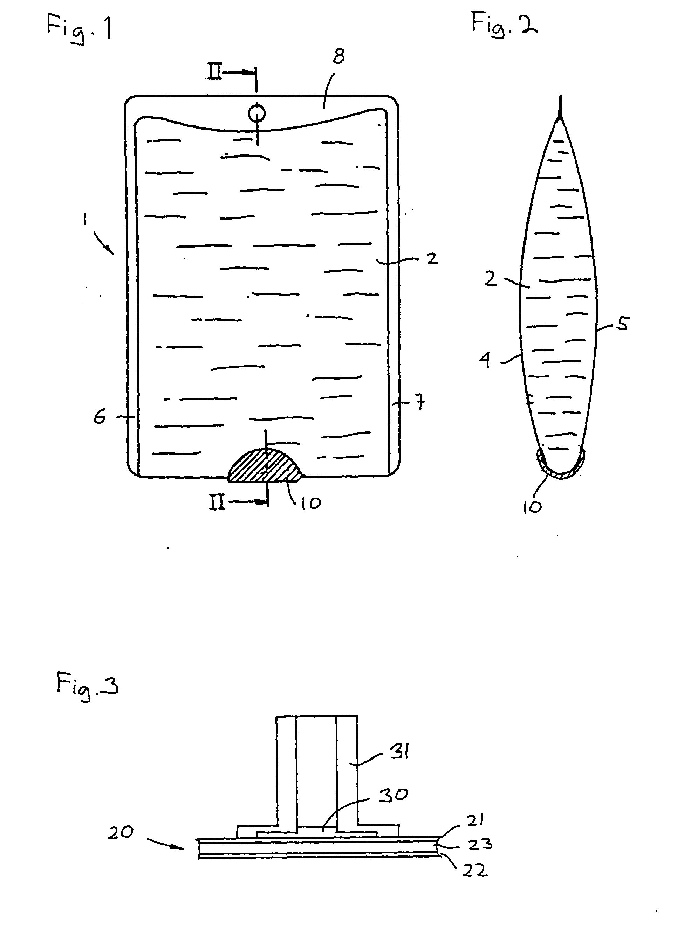

Fig. 1 shows a side view of an infusion bag which is generally denoted with 1. The infusion bag 1 exhibits an internal space 2 which is surrounded by walls 3 of a preferably flexible material, the material being penetratable by a pointed hypodermic needle. In the shown embodiment a piece of foil is folded upon itself corresponding to a lower edge 7 of the bag, thereby forming a front wall 4 connected to a back wall 5 along two opposite side edges 6, 7 and an upper edge 8. The connection can be accomplished by means of welding or, alternatively, gluing. In this manner, the internal space is formed between the joined edges and the lower folded edge. However, the invention is not limited to a special design of an infusion bag, but it can be manufactured in any way which is well-known to the skilled person. The foil may comprise one or more layers, e.g. for a saline solution the foil may be a single layer of polyethylene (PE).

The bag further comprises a connection means in the form of a septum member formed from a needle-penetratable self-sealing material, the septum member being formed from an elastomeric material allowing it to be welded directly onto the outer surface of the bag. In the shown embodiment the septum member is arranged at the lower rounded edge of the bag, however, it may be arranged at any desirable location just as the bag may be provided with additional connection means (not shown), e.g. a tubular member allowing an infusion set to be connected and held in place.

Fig. 3 shows a wall portion 20 of a further bag to which a septum member 30 formed from a needle-penetratable self-sealing material is welded. The wall is formed from a laminate comprising an outer weldable layer 21 allowing the septum member to be welded thereto, an intermediate layer 23 providing a barrier layer for an enclosed fluid, and an inner weldable layer 22 allowing the bag to be formed by welding. In addition to the septum a tubular member 31 is attached circumferentially in respect to the septum, preferably by welding, thereby providing a connection means for connecting e.g. an infusion set to the bag. The septum and the tube may be welded in the same process or independently of each other. The outer layer of the bag may be dispensed with expect for the portion to which the septum and the tubular member is attached.

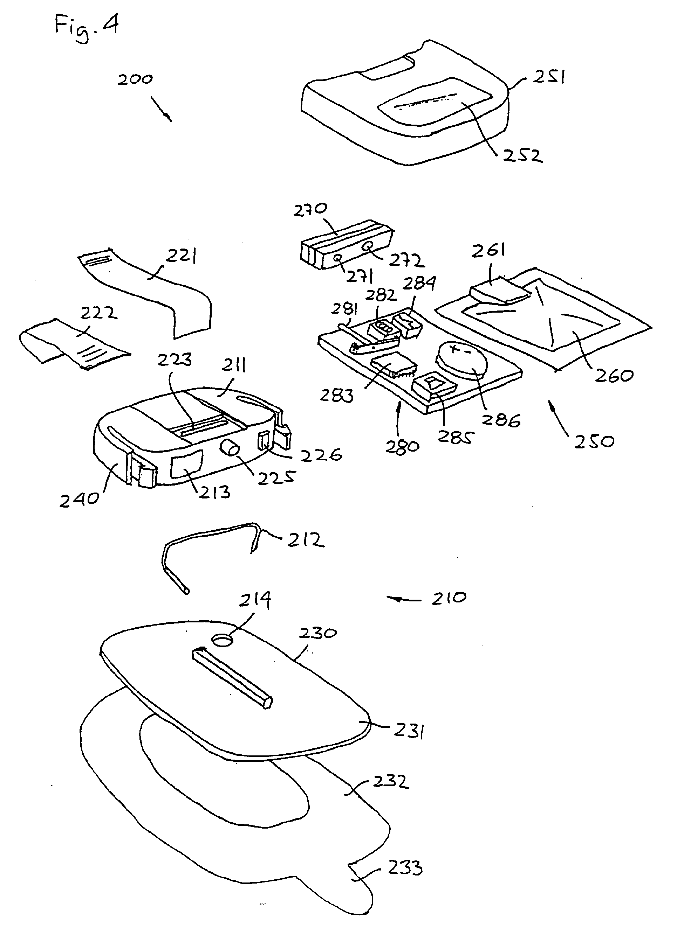

In fig. 4 an embodiment of a drug delivery device is shown, the device comprising a flexible reservoir allowing one or more aspects of the present invention to be implemented.

More specifically, fig. 4 shows in an exploded perspective view a medical device in the form of a drug delivery device 200 comprising a needle unit 210 having a needle housing 211, a base member 230 with a lower mounting surface adapted for application to the skin of a subject, and a separate reservoir and pump unit 250. In the shown embodiment the base member comprises a relatively rigid upper portion 231 attached to a more flexible adhesive patch member 232 provided with a gripable strip and having a lower adhesive surface providing the mounting surface *per se.* In the shown embodiment the needle housing is attached to the base plate as a separate unit, the two elements in combination forming the needle unit. Within the housing a hollow infusion needle 212 is pivotally arranged.

The needle unit comprises first and second openings 213, 214 which may be open or covered by needle penetratable membranes as described above with reference to fig. 14. The needle also has the same general configuration and pivotable arrangement as described above. The housing further comprises actuation means (not shown) for moving the needle between a retracted and extended state, and retraction means (not shown) for moving the needle between the extended and a retracted position. The actuation and retraction means are actuated by gripable first and second strip members 221, 222 connected to the respective means through slot-formed openings in the housing, of which the slot 223 for the first strip can be seen. The second strip is further connected to the patch strip 233. Arranged on the housing is user-actuatable male coupling means 240 in the form of a pair of resiliently arranged hook members adapted to cooperate with corresponding female coupling means on the reservoir unit. The housing further comprises connecting means 225 for establishing fluid communication between the pump unit and the reservoir (see below), and communication means 226 for activating and de-activating the expelling means.

The reservoir unit 250 comprises a housing 251 in which a reservoir and expelling means are arranged, the expelling means comprising a pump unit 270 and control and actuation means 280 therefore. The reservoir 260 is in the form of prefilled, flexible and collapsible pouch comprising a needle-penetratable septum 261 welded thereto and adapted to be arranged in fluid communication with the pump unit via pump inlet means 272. The reservoir is in the form of a flat pouch arranged substantially in parallel with the general plane of the device, the septum being mounted on the initially convex upper surface of the reservoir, thereby allowing a needle to be introduced therethrough substantially in parallel with the upper and lower walls of the reservoir. The housing comprises mounting means (not shown) allowing the septum to be fixated relative thereto, and a window 252 allowing the user to inspect the content of the reservoir. The shown pump is a mechanically actuated membrane pump, however, the expelling means may be of any suitable configuration.

The control and actuation means, which may be arranged on a PCB or flex-print, comprises a pump actuating member 281 in the form of a lever and piston arrangement driven by a coil actuator 282, a microprocessor 283 for controlling, among other, the pump actuation, a contact switch 284 cooperating with the communication means 226 on the needle unit, signal generating means 285 for generating an audible and/or tactile signal, and an energy source 286.

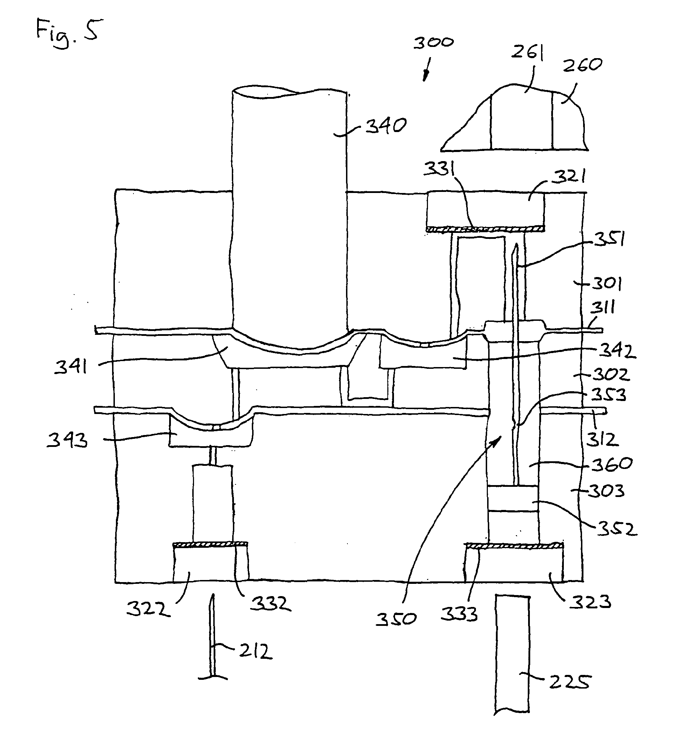

With reference to fig. 5 a pump unit 300 of a type suitable for use with the reservoir unit of fig. 4 is shown. The pump is a membrane pump comprising a piston-actuated pump membrane with flow-controlled inlet- and outlet-valves. The pump has a general three-layered construction comprising first, second and third members 301, 302, 303 between which are interposed first and second membrane layers 311, 312, whereby the inlet valve 342 and the pump membrane/pump chamber 341 is formed by the first and second members in combination with the first membrane layer, and the outlet valve 343 is formed by the second and third members in combination with the second membrane layer. The pump further comprises an inlet 321 and an outlet 322 as well as a connection opening 323 which are all three covered by respective membranes 331, 332, 333 sealing the interior of the pump in an initial sterile state. The inlet and outlet membranes are self-sealing, needle-penetratable septa (e.g. of a rubber-like material) and the connection membrane is a breakable membrane (e.g. made from paper). A fluid path is formed in the first member between the inlet and the inlet valve, fluid paths between the inlet valve, pump chamber and outlet valve is formed in the second member, and a fluid path between the outlet valve and the outlet is formed in the second member. The pump also comprises a piston 340 for actuating the pump membrane, the piston being driven by external driving means (not shown).

The pump further comprises a hollow connection needle 350 slidably positioned in a needle chamber 360 arranged behind the connection opening. The needle chamber is formed by the second and third members and comprises an internal septum through which the needle is arranged, the septum being formed by the first membrane layer and provides a seal between the chamber and the flow path. The needle comprises a pointed distal end 351, a proximal end on which is arranged a piston 352 and an intermediate opening 353 in flow communication with the distal end, the needle and the piston being slidably arranged relative to the internal septum and the chamber, respectively. As appears, the distal end of the needle is arranged outside the chamber protruding into the flow path.

In a situation of use the reservoir unit is attached to the reservoir unit (see fig. 15) whereby the proximal end of the infusion needle is introduced through the outlet septum 332 of the pump, and the connection member 225 is introduced through the connection membrane 333, thereby pushing the connection needle from its initial position to a position (not shown) in which the distal end is moved through the inlet membrane 331 and further through the needle-penetratable septum 261 of the reservoir, the outlet opening 353 is positioned in the flow path, thereby providing flow communication between the reservoir and the pump inlet.

As appears, when the two units are disconnected, the infusion needle 212 is withdrawn from the pump unit outlet whereas the connection needle permanently provides flow communication between the pump and the reservoir.

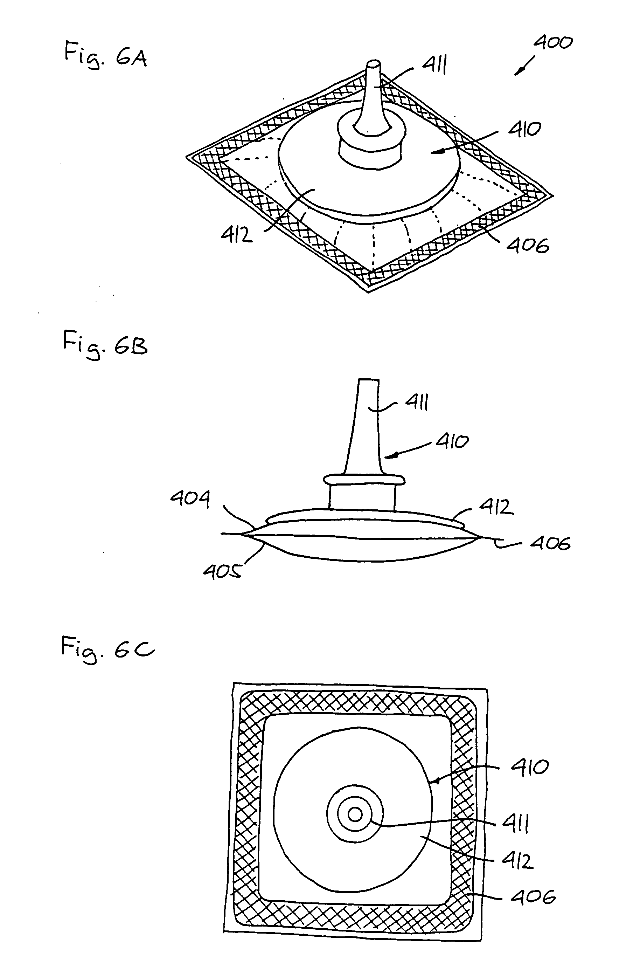

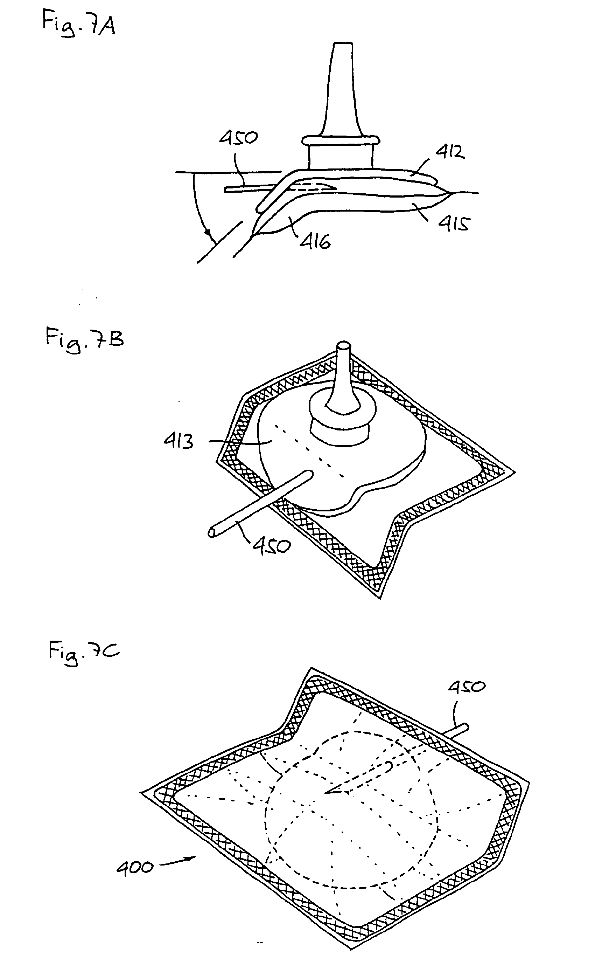

With reference to figs. 6A-6C and 7A-7C an alternative configuration of a flexible, prefilled drug reservoir suitable for use in a delivery device of the type shown in fig. 4.

The flexible reservoir 400 comprises first and second flexible wall foil members 404, 405 sealed together at the periphery thereof by welded seams 406, thereby forming a relatively flat pouch for containing the liquid drug, the welded seam of the pouch defining a general plane. An elastomeric septum member 410 is mounted on the first foil member, preferably by welding. The septum member comprises a relatively thin disc-formed base portion from the central portion of which projects an extension 411, the peripheral circumferential part 412 of the base portion forming a needle-penetratable self-sealing connection means. The projection may be utilized e.g. during manufacture and handling of the reservoir, as well as a mounting means for fixating the reservoir relative to other structures without having to interfere with movement of the flexible foil walls.

Although the filled reservoir 400 comprises a generally convex first surface allowing a needle to be inserted therethrough generally in parallel with the general plane of the reservoir, fig. 7A-C shows an arrangement of the reservoir allowing for improved insertion of a needle through the needle-penetratable portion 412 of the septum in parallel with the general plane of the reservoir.

More specifically, by bending or deflecting a portion of the reservoir downwardly relative to the general plane (by e.g. approximately 30 degrees as shown in fig. 7A), an area 413 of the needle-penetratable portion of the septum is "presented" to a needle 450 arranged in parallel with the general plane of the reservoir, thereby allowing for ease of insertion. As shown in fig. 7A, the bend reservoir comprises a first "major" portion 415 being arranged substantially corresponding to the general plane, and a second "minor" portion 416 being deflected relative thereto in a direction away from the first surface, such that a part 413 of the peripheral portion of the septum member is arranged on the second portion of the reservoir. In figs. 7A-7C the reservoir is shown without the means for bending the reservoir, however, such means can be provided by structures of a surrounding housing.

Example: A reservoir containing 3 ml of insulin was manufactured from two foil members of a three-layered laminate comprising an intermediate layer of PCTFE co-extruded with epoxy modified polyethylene imine (a tie-layer) and an inner layer of PE, and with an outer layer of PP laminated on the PCTFE layer. A septum member made from a thermoplastic elastomeric rubber-compound was welded to the outer PP layer before the two foil members were welded to each other along the peripheries thereof, the insulin being filled into the reservoir before it was completely sealed.

In the above description of the preferred embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A container (1, 260, 400) adapted to contain a fluid, comprising:
- a first wall portion formed from a needle-penetratable material, the first wall portion comprising an outer first mounting surface,
- a septum member (10, 261, 410) formed from a needle-penetratable self-sealing material, the septum member comprising a second mounting surface,
- wherein the septum member, corresponding to the second mounting surface, has been mounted on the first mounting surface by means of welding together the two surfaces.

2. A container as defined in claim 1, wherein the first wall portion is made from a single layer of material.

3. A container as defined in claim 1, wherein the container generally is formed from a single material or singly type of laminate.

4. A container as defined in claim 1, wherein the container has an outer surface generally formed from a first material and an inner surface generally formed from a second material.

5. A container as defined in claim 1, comprising at least first and second flexible foil members (4, 5, 404, 405) sealed together to form at least one enclosed cavity for containing the fluid.

6. A container as defined in claim 5, wherein the foil members are composite laminates of continuous layers including an outer layer (21) and an inner layer (22).

7. A container as defined in claim 6, wherein the foil members comprise an intermediate layer (23), the inner layer being formed from a weldable material, the foil members being sealed together at least partially by welding.

8. A container as defined in any of the previous claims, wherein at least a portion of the container is transparent or translucent such that a contained fluid can be viewed therethrough.

9. A container as defined in any of claims 5-8, wherein at least a portion (412) of the septum member (410) is configured to flex together with the portion of the container to which it is mounted.

10. A container as defined in claim 9, wherein the septum member comprises a first relatively thin needle-penetratable portion (412) and a second portion (411) projecting from the portion of the container to which the septum is mounted.

11. A container as defined in any of the previous claims, wherein the first mounting surface is formed from a material selected from a first group of materials comprising PE, PP, OPP, PET, OPET, PA and OPA , and the second mounting surface is formed from a material selected from a second group of materials comprising thermoplastic elastomeric materials.

12. A container device (200), comprising:
- a container (260, 400) as defined in claim 1 wherein the septum member serves as a container outlet means,
- a needle (350, 450) having a generally straight inlet portion and an outlet, the inlet being adapted to be arranged in fluid communication with the reservoir,
- wherein the needle and the container are moveable relative to each other from an initial position in which there is no fluid communication therebetween and a connected position in which the needle inlet is arranged in fluid communication with the container through the reservoir outlet means, and
- wherein the needle in the connected position penetrates the septum member at an inclined angle relative to the first wall portion.

13. A container device as defined in claim 12, wherein the container comprises first and second flexible foil members (404, 405) sealed together to form an enclosed cavity for containing the fluid, the first foil member comprising the first surface, the container having a pouch-like configuration defining a general plane, the needle (450) in the connected position being arranged substantially in parallel with the general plane.

14. A container device comprising:
- a housing (251), and
- a container as defined in claim 1 (260) arranged in the housing,
- wherein the septum member is mounted to the housing.

15. A container device as defined in claim 14, wherein the container is flexible having a pouch-like configuration, substantially the entire flexible container apart from the septum member being free to move relative to the housing.
